(19) Europäisches Patentamt
European Patent Office
Office européen des brevets

(11) Veröffentlichungsnummer: **0 344 684**
**A2**

(12) # EUROPÄISCHE PATENTANMELDUNG

(21) Anmeldenummer: **89109649.7**

(51) Int. Cl.⁴: **C07D 237/16 , A01N 47/06**

(22) Anmeldetag: **29.05.89**

(30) Priorität: **03.06.88 DE 3818848**

(43) Veröffentlichungstag der Anmeldung:
**06.12.89 Patentblatt 89/49**

(84) Benannte Vertragsstaaten:
**CH DE FR GB IT LI**

(71) Anmelder: **HOECHST AKTIENGESELLSCHAFT**
**Postfach 80 03 20**
**D-6230 Frankfurt am Main 80(DE)**

(72) Erfinder: **Lüders, Walter, Dr.**
**Feldbergstrasse 16**
**D-6056 Heusenstamm(DE)**
Erfinder: **Salbeck, Gerhard, Dr.**
**Frankfurter Strasse 34**
**D-6238 Hofheim am Taunus(DE)**
Erfinder: **Bauer, Klaus, Dr.**
**Doorner Strasse 53D**
**D-6450 Hanau(DE)**
Erfinder: **Bieringer, Hermann, Dr.**
**Eichenweg 26**
**D-6239 Vockenhausen(DE)**

(54) **Pyridazinonderivate, Verfahren zu ihrer Herstellung, sie enthaltende Mittel und ihre Verwendung im Pflanzenschutz.**

(57) Gegenstand der vorliegenden Erfindung sind daher Mittel zum Schutz von Kulturpflanzen gegen phytotoxische Nebenwirkungen von Herbiziden, dadurch gekennzeichnet, daß sie eine Verbindung der Formel I

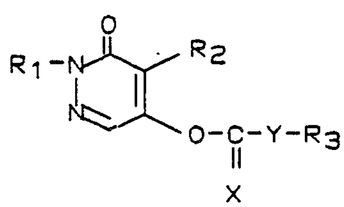

(I),

worin

$R_1$ Alkyl, Alkoxyalkyl, Cyanoalkyl, Aryl, das ein- oder mehrfach durch Alkyl, Halogen, Haloalkyl, Alkoxy, Phenyl, Phenoxy oder Nitro substituiert sein kann, Aryl alkyl, Aryloxyalkyl, wobei der Arylrest jeweils ein- oder mehrfach durch Alkyl, Halogen, Haloalkyl, Haloalkyl, Alkoxy, Aroyl, Arylthio oder Nitro substituiert sein kann, oder Pyridyl

$R_2$ Halogen oder Alkyl,

X Sauerstoff oder Schwefel

Y Sauerstoff oder Schwefel und

$R_3$ Alkyl, das ein- oder mehrfach durch Halogen oder Alkoxy substituiert sein kann, Alkenyl, Cycloalkyl, Aryl, das ein- oder mehrfach durch Alkyl, Halogen, Alkoxy, Haloalkyl, Haloalkoxy oder Nitro substituiert sein kann, Arylalkyl, Aryloxyalkyl, wobei der Arylteil jeweils durch Halogen oder Nitro substituiert sein kann, bedeuten, in Kombination mit einem Herbizid enthalten.

Die Verbindungen der Formel I sind zum Teil neu. Ferner wird ein Verfahren zu ihrer Herstellung beschrieben.

EP 0 344 684 A2

**Pyridazinonderivate, Verfahren zu ihrer Herstellung, sie enthaltende Mittel und ihre Verwendung im Pflanzenschutz**

Gegenstand der vorliegenden Erfindung sind Pyridazinonderivate, Verfahren zu ihrer Herstellung, sie enthaltende Mittel und ihre Verwendung im Pflanzenschutz

Aus der CS-PS 132510 sind bestimmte Pyridazinonderivate und deren Verwendung als Fungizide bekannt.

Es wurde nun gefunden, daß eine größere Gruppe von Pyridazinonderivaten die Eigenschaft besitzt, phytotoxische Nebenwirkungen von Pflanzenschutzmitteln, insbesondere von Herbiziden, beim Einsatz in Nutzpflanzenkulturen zu vermindern oder ganz auszuschalten.

Gegenstand der vorliegenden Erfindung sind daher Mittel zum Schutz von Kulturpflanzen gegen phytotoxische Nebenwirkungen von Herbiziden, dadurch gekennzeichnet, daß sie eine Verbindung der Formel I

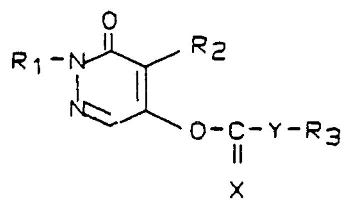 (I),

worin

$R_1$ $(C_1$-$C_6)$-Alkyl, $(C_1$-$C_3)$-Alkoxy-$(C_1$-$C_3)$-alkyl, Cyano-$(C_1$-$C_3)$-alkyl, Aryl, das ein- oder mehrfach durch $(C_1$-$C_4)$-Alkyl, Halogen, $(C_1$-$C_3)$-Haloalkyl, $(C_1$-$C_4)$-Alkoxy, Phenyl, Phenoxy oder Nitro substituiert sein kann, Aryl-$(C_1$-$C_3)$-alkyl, Aryloxy-$(C_1$-$C_3)$-alkyl, wobei der Arylrest jeweils ein- oder mehrfach durch $(C_1$-$C_4)$- Alkyl, Halogen, $(C_1$-$C_3)$-Haloalkyl, $(C_1$-$C_3)$-Haloalkyl, $(C_1$-$C_4)$-Alkoxy, Aroyl, Arylthio oder Nitro substituiert sein kann, oder Pyridyl

$R_2$ Halogen oder $(C_1$-$C_4)$Alkyl,

X Sauerstoff oder Schwefel

Y Sauerstoff oder Schwefel und

$R_3$ $(C_1$-$C_{18})$-Alkyl, das ein- oder mehrfach durch Halogen oder $(C_1$-$C_4)$-Alkoxy substituiert sein kann, $(C_2$-$C_4)$-Alkenyl, $(C_3$-$C_8)$-Cycloalkyl, Aryl, das ein- oder mehrfach durch $(C_1$-$C_3)$-Alkyl, Halogen, $(C_1$-$C_3)$-Alkoxy, $(C_1$-$C_3)$-Haloalkyl, $(C_1$-$C_3)$-Haloalkoxy oder Nitro substituiert sein kann, Aryl-$(C_1$-$C_3)$-alkyl, Aryloxy-$(C_1$-$C_3)$-alkyl, wobei der Arylteil jeweils durch Halogen oder Nitro substituiert sein kann, bedeuten, in Kombination mit einem Herbizid enthalten.

Dabei steht der Rest Alkyl für geradkettiges oder verzweigtes Alkyl, Aryl bedeutet bevorzugt Phenyl und Halogen bevorzugt Cl oder Br.

(1) Bevorzugt sind die Verbindungen der Formel I, worin

$R_1$ $(C_1$-$C_4)$-Alkyl oder Aryl, das durch Halogen substituiert sein kann, und

$R_3$ $(C_1$-$C_{12})$-Alkyl, das ein- oder mehrfach durch Halogen substituiert sein kann, oder Benzyl

bedeuten.

2) Besonders bevorzugt sind die Verbindungen der Formel I, worin

$R_1$ $(C_1$-$C_4)$-Alkyl

$R_2$ Chlor

X Sauerstoff und

$R_3$ $(C_1$-$C_8)$-Alkyl, das durch Halogen substituiert sein kann,

bedeuten.

Die Verbindungen der Formel I, bei denen entweder X oder Y oder X und Y Schwefel bedeuten (Ia), sind neu und ebenfalls Gegenstand der vorliegenden Erfindung. Bevorzugt sind auch hier (Ia) die oben unter 1) und 2) genannten Verbindungen.

Von den Verbindungen der Formel I, bei denen X und Y Sauerstoff bedeuten, sind die Verbindungen der Formel Ib, bei denen

$R_1$ = tert. Butyl

$R_2$ = Halogen oder $(C_1$-$C_4)$Alkyl und

$R_3$ = ($C_1$-$C_4$)-Haloalkyl oder ($C_1$-$C_{12}$)-Alkyl bedeuten, neu und Gegenstand der vorliegenden Erfindung.

Besonders bevorzugt unter diesen Verbindungen sind diejenigen, bei denen

$R_1$ = tert. Butyl

$R_2$ = Cl oder Br und

$R_3$ = ($C_1$-$C_4$)-Haloalkyl oder ($C_3$-$C_8$)-Alkyl bedeuten.

Ferner umfaßt die vorliegende Erfindung ein Verfahren zur Herstellung von Verbindungen der Formeln Ia und Ib, dadurch gekennzeichnet, daß man eine Verbindung der Formel II

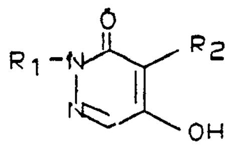

(II),

worin

$R_1$ und $R_2$ die Bedeutungen wie in Formeln Ia und Ib besitzen, mit einer Verbindung der Formel III

$Z-\underset{\underset{X}{\|}}{C}-Y-R_3$ (III),

worin

Z = Halogen bedeutet und X, Y und $R_3$ die Bedeutungen wie in Formeln Ia und Ib besitzen, in Gegenwart eines säurebindenden Mittels umsetzt.

Die Herstellung der Verbindungen der Formeln Ia und Ib kann auch dadurch erfolgen, daß man ein Alkali-, Ammonium- oder Erdalkalisalz der Pyridazinonderivate der Formel II mit einer Verbindung der Formel III ohne Zusatz eines Säureakzeptors umsetzt.

Die als Ausgangsstoffe zu verwendenden Pyridazinonderivate der Formel II sind literaturbekannt bzw. können nach bekannten Verfahren hergestellt werden (siehe Angew. Chemie 77, 1965, S. 282, sowie dort zitierte Literatur). Sie sind durch die Formel II allgemein definiert. Vorzugsweise stehen jedoch in der Formel II $R_1$ für einen geradkettigen oder verzweigten Alkylrest mit 1 bis 4 Kohlenstoffatomen und $R_2$ für Chlor oder Brom.

Als Beispiele für Ausgangsstoffe der Formel II können genannt werden für den Rest

$R_1$ eine Methyl-, Ethyl-, n-Propyl-, Isopropyl-, n-Butyl-, sek.-Butyl- oder tert. Butylgruppe, für den Rest

$R_2$ Chlor, Brom, Methyl, Ethyl, n-Propyl, iso-Propyl oder n-Butyl.

Die als Ausgangsstoffe zu verwendenden Susbtanzen der Formel III sind allgemein bekannt und im Handel erhältlich. Als Beispiele für diese Ausgangsstoffe können genannt werden:

Chlorkohlensäure-methylester, -ethylester, -n-propylester, -isopropylester, -n-butylester, -sek.butylester, -isobutylester, -1-methylbutylester, -1-ethylpropylester, -n-pentylester, -isopentylester, -1-methylisobutylester, -neopentylester, n-hexylester, -1-methylpentylester, -1-ethylbutylester, -1,2-dimethylbutylester, -1,3-dimethylbutylester, -2-ethylbutylester, -1-ethylisobutylester, -n-heptylester, -1-ethylpentylester, -1-propylbutylester,-1-ethylisopentylester, -1-methyl-2-ethylbutylester, -1-isopropyl-2-methylpropylester, -n-octylester, -1-methylheptylester, -2-ethylhexylester, 2,2-diethylbutylester, -1-isobutyl-3-methylbutylester, -1-tert.-butyl-2,2-dimethylpropylester, -n-nonylester, -n-decylester, -n-undecylester, -n-dodecylester, -n-tetradecylester, -n-hexadecylester, -n-octadecylester, -allylester, -1-methylallylester, -but-2-enylester, -2-methoxyethylester, 2-ethoxyethylester, -2-propoxyethylester, -2-butoxyethylester, -2-fluorethylester, -2-chlorethylester, -2-bromethylester, -3-chlorpropylester, -2-chlor-1-methylethylester, -3-chlor-1-ethylpropylester, -1,2-dichlorethylester, -1,2-dibromethylester, -2-chlor-1-chlormethylethylester, -1,1,2-trichlorethylester, -2,2,2-trichlorethylester, -2,2,2-tribromethylester, -1,1,2,2-tetrachlorethylester, -1,2,2,2-tetrachlorethylester, -1-dichlormethyl-1,2,2-trichlorethylester, -2,3-dichlorpropylester, -2,3-dibrompropylester, -2,2-dichlor-1-chlormethylethylester, -2-chlor-1-methylpropylester, -2-chlor-2-methylpropylester, -4-chlorbutylester, -5-chlorpentylester, -6-chlorhexylester, -8-chloroctylester, -10-chlordecylester, -3-chlorallylester, -1-chlormethylallylester, -cyclohexylester, menthylester, -bornylester, -phenylester, -o-tolylester, -m-tolylester, p-tolylester, -2,4-dimethylphenylester, -3-methyl-4-isopropylphenylester, -2-chlorphenylester, -4-chlorphenylester, -4-bromphenylester, -2,4,6-tribromphenylester, -2-methoxyphenylester, -2-methoxy-4-propenylphenylester, 2-methoxy-4-allylphenylester, -2-nitrophenylester, -3-nitrophenylester, -4-nitrophenylester, -alpha-naphthylester, -beta-naphthylester, -benzylester, -4-nitrobenzylester, -2-phenethylester, -2-phenoxyethylester;

Chlorthiokohlensäure-S-methylester, -S-ethylester, -S-propylester, -S-isopropylester, -S-n-butylester, -S-isobutylester, -S-tert.-butylester, -S-hexylester, -S-dodecylester, -S-2-chlorethylester, -S-2-chlorpropylester, -S-3-chlorpropylester, -S-allylester, -S-phenylester, -S-beta-naphthylester, -S-4-chlorphenylester, -S-pentafluorphenylester, -S-4-methoxyphenylester, -S-benzylester, -O-methylester, -O-ethylester, -O-propylester, -O-

isopropylester, -O-butylester, -O-2-chlorethylester, -O-phenylester, -O-o-tolyester, -O-m-tolylester, -O-p-tolylester, -O-4-tert.-butylphenylester, -O-2,6-dimethylphenylester, -O-2-chlorphenylester, -O-3-chlorphenylester, -O-4-chlorphenylester, -O-2,4-dichlorphenylester, -O-3,4-dichlorphenylester, -O-2-bromphenylester, -O-2,4,5-tribromphenylester, -O-alpha-naphthylester, -O-betanaphthylester, -O-4-nitrophenylester, -O-2-chlor-4-nitrophenylester, -O-benzylester, -O-2-biphenylester, -O-3-biphenylester, Dithiokohlensäurechlorid-methylester, -ethylester, -propylester, -isopropylester, -butylester, -phenylester, -4-nitrophenylester, -o-tolylester, -p-tolylester, -2-biphenylester, -benzylester.

Das Verfahren zur Herstellung der erfindungsgemäßen Verbindungen wird bevorzugt unter Mitverwendung geeigneter Lösungs- und Verdünnungsmittel durchgeführt. Hierzu eignen sich beispielsweise aliphatische und aromatische, gegebenenfalls chlorierte Kohlenwasserstoffe wie Methylenchlorid, Chloroform, Tetrachlorkohlenstoff, 1,2-Dichlorethan, Toluol, Xylol, Chlorbenzol, Ether wie Diethyl-, Diisopropyl- oder Glykoldimethylether, Ketone wie Aceton, Methylethylketon, Nitrile wie Acetonitril oder Propionitril oder Amide wie N,N-Dimethylformamid. Gegebenenfalls können Gemische dieser Lösungsmittel oder Gemische dieser Lösungsmittel mit Wasser, gegebenenfalls unter Zusatz eines Phasentransferkatalysators wie Benzyltriethylammoniumchlorid eingesetzt werden. Als säurebindende Mittel können dem Reaktionsgemisch anorganische Basen wie beispielsweise Natriumhydroxid, Kaliumhydroxid, Natriumcarbonat, Kaliumcarbonat, Natriumbicarbonat oder organische Basen wie Triethylamin, Kalium-tert.-butylat, Pyridin, N,N-Dimethylanilin zugesetzt werden.

Die Reaktion findet im allgemeinen zwischen Raumtemperatur und der Siedetemperatur des eingesetzten Lösungsmittels statt.

Die Ausgangsstoffe werden im allgemeinen in stöchiometrischen Mengen eingesetzt.

Die Isolierung der Reaktionsprodukte erfolgt nach allgemein üblichen Methoden, z.B. durch Abdampfen des Lösungsmittels, gegebenenfalls unter vermindertem Druck oder durch Verteilen des Rohproduktes zwischen zwei Phasen und die sich daran anschließende übliche Aufarbeitung. Zur Charakteriseriung dienen Brechungsindex oder Schmelzpunkt.

Bei der Anwendung von Pflanzenbehandlungsmitteln, insbesondere von Herbiziden, können unerwünschte, nicht tolerierbare Schäden an Kulturpflanzen auftreten. Besonders bei der Applikation von Herbiziden nach dem Auflaufen der Kulturpflanzen besteht daher oft das Bedürfnis, das Risiko einer möglichen Phytotoxizität zu vermeiden.

Überraschenderweise wurde gefunden, daß Verbindungen der Formel I die Eigenschaften haben, phytotoxische Nebenwirkungen von Pflanzenschutzmitteln, insbesondere von Herbiziden, beim Einsatz in Nutzpflanzenkulturen zu vermindern oder ganz auszuschalten. Die Verbindungen der Formel I sind in der Lage, schädliche Nebenwirkungen der Herbizide völlig aufzuheben, ohne die Wirksamkeit dieser Herbizide gegen Schadpflanzen zu schmälern.

Solche Verbindungen, die die Eigenschaften besitzen, Kulturpflanzen gegen phytotoxische Schäden durch Herbizide zu schützen, ohne die eigentliche herbizide Wirkung dieser Mittel zu beeinträchtigen, werden "Antidote" oder "Safener" genannt.

Das Einsatzgebiet herkömmlicher Herbizide kann durch Zugabe der Safenerverbindung der Formel I ganz erheblich vergrößert werden.

Gegenstand der vorliegenden Erfindung ist daher auch ein Verfahren zum Schutz von Kulturpflanzen gegen phytotoxische Nebenwirkungen von Pflanzenschutzmitteln, insbesondere Herbiziden, das dadurch gekennzeichnet ist, daß man die Pflanzen, Pflanzensamen oder Anbauflächen mit einer Verbindung der Formel I vor, nach oder gleichzeitig mit dem Pflanzenschutzmittel behandelt.

Herbizide, deren phytotoxische Nebenwirkungen mittels der Verbindungen der Formel I herabgesetzt werden können, sind z.B. Carbamate, Thiocarbamate, Halogenacetanilide, substituierte Phenoxy-, Naphthoxy- und Phenoxyphenoxy-carbonsäurederivate sowie Heteroaryloxyphenoxycarbonsäurederivate wie Chinolyloxy-, Chinoxalyloxy, Pyridyloxy-, Benzoxazolyloxy-, Benzthiazolyloxy-phenoxy-carbonsäureester und ferner Dimedonoximabkömmlinge. Bevorzugt hiervon sind Phenoxyphenoxy- und Heteroaryloxyphenoxy-carbonsäureester. Als Ester kommen hierbei insbesondere niedere Alkyl-, Alkenyl- und Alkinylester in Frage.

Beispielsweise seien, ohne daß dadurch eine Beschränkung erfolgen soll, folgende Herbizide genannt:

A) Herbizide vom Typ der Phenoxyphenoxy- und Heteroaryloxyphenoxycarbonsäure-($C_1$-$C_4$)-Alkyl-, ($C_2$-$C_4$)-Alkenyl- oder ($C_3$-$C_4$)-Alkinylester wie
2-(4-(2,4-Dichlorphenoxy)-phenoxy)-propionsäuremethylester,
2-(4-(4-Brom-2-chlorphenoxy)-phenoxy)-propionsäuremethylester,
2-(4-(4-Trifluormethylphenoxy)-phenoxy)-propionsäuremethylester,
2-(4-(2-Chlor-4-trifluormethylphenoxy)-phenoxy)-propionsäuremethylester,
2-(4-(2,4-Dichlorbenzyl)-phenoxy)-propionsäuremethylester, 2-Isopropylideneamino-oxyethyl(R)-2-[4-(6-

| Verb.-Nr. | $R_1$ | $R_2$ | X | Y | $R_3$ | Fp. (°C) $n_D^{23}$ |
|---|---|---|---|---|---|---|
| 18 | -C(CH3)3 | Cl | O | O | -n-C3H7 | 1,5043 |
| 19 | -C(CH3)3 | Cl | O | O | -CH(CH3)2 | 1,5133 |
| 20 | -C(CH3)3 | Cl | O | O | -n-C4H9 | 1,528 |
| 21 | -C(CH3)3 | Cl | O | O | -CH2-CH(CH3)2 | 1,5 |
| 22 | -C(CH3)3 | Cl | O | O | -CH(CH3)-CH2-CH3 | |
| 23 | -C(CH3)3 | Cl | O | O | -CH(CH3)-CH2-CH2-CH3 | |
| 24 | -C(CH3)3 | Cl | O | O | -CH(C2H5)-CH2-CH3 | |
| 25 | -C(CH3)3 | Cl | O | O | -n-C5H11 | |
| 26 | -C(CH3)3 | Cl | O | O | -CH(CH3)-CH2-CH(CH3)2 | |
| 27 | -C(CH3)3 | Cl | O | O | -CH2-CH2-CH(CH3)2 | |
| 28 | -C(CH3)3 | Cl | O | O | -CH2-C(CH3)3 | |
| 29 | -C(CH3)3 | Cl | O | O | -n-C6H13 | 1,4934 |
| 30 | -C(CH3)3 | Cl | O | O | -CH(CH3)-(CH2)3-CH3 | |
| 31 | -C(CH3)3 | Cl | O | O | -CH(C2H5)-(CH2)2-CH3 | |
| 32 | -C(CH3)3 | Cl | O | O | -C(CH3)2-CH2-CH2-CH3 | |
| 33 | -C(CH3)3 | Cl | O | O | -CH(CH3)-CH(CH3)-CH2-CH3 | |
| 34 | -C(CH3)3 | Cl | O | O | -CH2-CH(C2H5)-CH2-CH3 | |
| 35 | -C(CH3)3 | Cl | O | O | -CH(C2H5)-CH2-CH(CH3)2 | |
| 36 | -C(CH3)3 | Cl | O | O | -n-C7H15 | |
| 37 | -C(CH3)3 | Cl | O | O | -CH(C2H5)-(CH2)3-CH3 | |
| 38 | -C(CH3)3 | Cl | O | O | -CH(C3H7)-(CH2)2-CH3 | |
| 39 | -C(CH3)3 | Cl | O | O | -CH(CH3)-CH(C2H5)-CH2-CH3 | |
| 40 | -C(CH3)3 | Cl | O | O | -n-C8H17 | 1,548 |
| 41 | -C(CH3)3 | Cl | O | O | -CH(CH3)-(CH2)5-CH3 | |
| 42 | -C(CH3)3 | Cl | O | O | -CH2-CH(C2H5)-(CH2)3-CH3 | 1,584 |
| 43 | -C(CH3)3 | Cl | O | O | -CH2-C(C2H5)2-CH2-CH3 | |

| Verb.-Nr. | $R_1$ | $R_2$ | X | Y | $R_3$ | Fp. (°C) $n_D^{23}$ |
|---|---|---|---|---|---|---|
| 44 | -C(CH3)3 | Cl | O | O | -n-C9H19 | |
| 45 | -C(CH3)3 | Cl | O | O | -n-C10H21 | 1,5115 |
| 46 | -C(CH3)3 | Cl | O | O | -n-C11H23 | |
| 47 | -C(CH3)3 | Cl | O | O | -n-C12H25 | |
| 48 | -C(CH3)3 | Cl | O | O | -n-C14H29 | |
| 49 | -C(CH3)3 | Cl | O | O | -n-C16H33 | |
| 50 | -C(CH3)3 | Cl | O | O | -n-C18H37 | |
| 51 | -C(CH3)3 | Cl | O | O | -CH2-CH=CH2 | 1,5432 |
| 52 | -C(CH3)3 | Cl | O | O | -CH(CH3)-CH=CH2 | |
| 53 | -C(CH3)3 | Cl | O | O | -CH2-CH=CH-CH3 | |
| 54 | -C(CH3)3 | Cl | O | O | -CH2-CH2-O-CH3 | |
| 55 | -C(CH3)3 | Cl | O | O | -CH2-CH2-O-CH2-CH3 | |
| 56 | -C(CH3)3 | Cl | O | O | -CH2-CH2-O-C3H7 | |
| 57 | -C(CH3)3 | Cl | O | O | -CH2-CH2-O-C4H9 | |
| 58 | -C(CH3)3 | Cl | O | O | -CH2-CH2-F | 1,51 |
| 59 | -C(CH3)3 | Cl | O | O | -CH2-CH2-Cl | 1,524 |
| 60 | -C(CH3)3 | Cl | O | O | -CH2-CH2-Br | 1,5347 |
| 61 | -C(CH3)3 | Cl | O | O | -CH2-CH2-CH2Cl | |
| 62 | C(CH3)3 | Cl | O | O | -CH(CH3)-CH2Cl | |
| 63 | -C(CH3)3 | Cl | O | O | -CH(C2H5)-CH2-CH2Cl | |
| 64 | -C(CH3)3 | Cl | O | O | -CHCl-CH2Cl | |
| 65 | -C(CH3)3 | Cl | O | O | -CHBr-CH2Br | |
| 66 | -C(CH3)3 | Cl | O | O | -CH(CH2Cl)-CH2Cl | |
| 67 | -C(CH3)3 | Cl | O | O | -CCl2-CH2Cl | |
| 68 | -C(CH3)3 | Cl | O | O | -CH2-CCl3 | 113 |

| Verb.-Nr. | R1 | R2 | X | Y | R3 | Fp. (°C) $n_D^{23}$ |
|---|---|---|---|---|---|---|
| 69 | -C(CH3)3 | Cl | O | O | -CH2-CBr3 | |
| 70 | -C(CH3)3 | Cl | O | O | -CCl2-CHCl2 | |
| 71 | -C(CH3)3 | Cl | O | O | -CHCl-CCl3 | |
| 72 | -C(CH3)3 | Cl | O | O | -CH(CH2Cl)-CHCl2 | |
| 73 | -C(CH3)3 | Cl | O | O | -CCl(CHCl2)-CHCl2 | |
| 74 | -C(CH3)3 | Cl | O | O | -CH2-CHCl-CH2Cl | 1,529 |
| 75 | -C(CH3)3 | Cl | O | O | -CH2-CHBr-CH2Br | 1,579 |
| 76 | -C(CH3)3 | Cl | O | O | -CH(CH3)-CH2-CH2Cl | |
| 77 | -C(CH3)3 | Cl | O | O | -CH2-CCl(CH3)-CH3 | |
| 78 | -C(CH3)3 | Cl | O | O | -CH2-CH2-CH2-CH2Cl | |
| 79 | -C(CH3)3 | Cl | O | O | -(CH2)4-CH2Cl | |
| 80 | -C(CH3)3 | Cl | O | O | -(CH2)5-CH2Cl | |
| 81 | -C(CH3)3 | Cl | O | O | -(CH2)7-CH2Cl | |
| 82 | -C(CH3)3 | Cl | O | O | -(CH2)9-CH2Cl | |
| 83 | -C(CH3)3 | Cl | O | O | -CH2-CH=CHCl | |
| 84 | -C(CH3)3 | Cl | O | O | 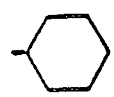 | 1,492 |
| 85 | -C(CH3)3 | Cl | O | O | 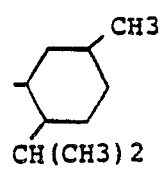 | 1,5022 |
| 86 | -C(CH3)3 | Cl | O | O | 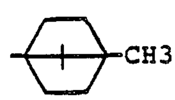 | |
| 87 | -C(CH3)3 | Cl | O | O | 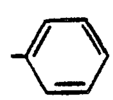 | 117 |

| Verb.-Nr. | $R_1$ | $R_2$ | X | Y | $R_3$ | Fp. (°C) $n_D^{23}$ |
|---|---|---|---|---|---|---|
| 88 | -C(CH3)3 | Cl | O | O | 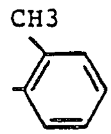 | |
| 89 | -C(CH3)3 | Cl | O | O | 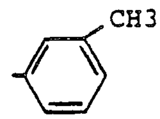 | |
| 90 | -C(CH3)3 | Cl | O | O | 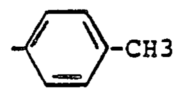 | |
| 91 | -C(CH3)3 | Cl | O | O | 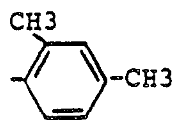 | |
| 92 | -C(CH3)3 | Cl | O | O | 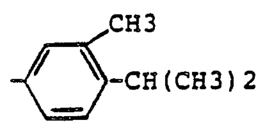 | |
| 93 | -C(CH3)3 | Cl | O | O | 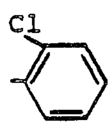 | |
| 94 | -C(CH3)3 | Cl | O | O | 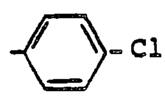 | |
| 95 | -C(CH3)3 | Cl | O | O | 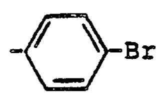 | |
| 96 | -C(CH3)3 | Cl | O | O | 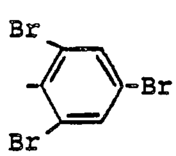 | |
| 97 | -C(CH3)3 | Cl | O | O | 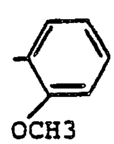 | |

| Verb.-Nr. | $R_1$ | $R_2$ | X | Y | $R_3$ | Fp. (°C) $n_D^{23}$ |
|---|---|---|---|---|---|---|
| 98 | -C(CH3)3 | Cl | O | O | 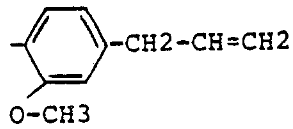 | |
| 99 | -C(CH3)3 | Cl | O | O | 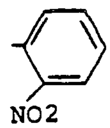 | |
| 100 | -C(CH3)3 | Cl | O | O | 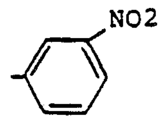 | |
| 101 | -C(CH3)3 | Cl | O | O | 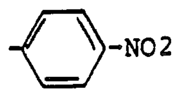 | 119-121 |
| 102 | -C(CH3)3 | Cl | O | O | 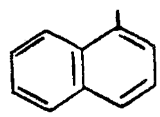 | |
| 103 | -C(CH3)3 | Cl | O | O | 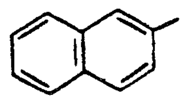 | |
| 104 | -C(CH3)3 | Cl | O | O | 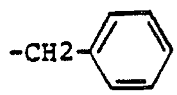 | 1,556 |
| 105 | -C(CH3)3 | Cl | O | O | 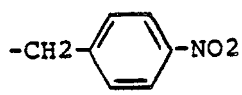 | 107-109 |
| 106 | -C(CH3)3 | Cl | O | O | 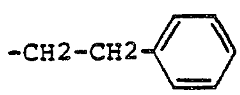 | |
| 107 | -C(CH3)3 | Cl | O | O | 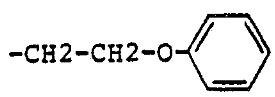 | |
| 108 | -C(CH3)3 | Cl | O | O | 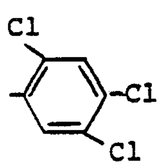 | 155 |

| Verb.-Nr. | $R_1$ | $R_2$ | X | Y | $R_3$ | Fp. (°C) $n_D^{23}$ |
|---|---|---|---|---|---|---|
| 109 | -C(CH3)3 | Cl | O | O | -C(CH3)2-CCl3 | |
| 110 | -C(CH3)3 | Br | O | O | -C2H5 | |
| 111 | -C(CH3)3 | Br | O | O | -CH2-CH(CH3)2 | 1,5106 |
| 112 | -C(CH3)3 | Br | O | O | -CH2-CH2Br | 1,579 |
| 113 | -CH2-CH2CN | Cl | O | S | -C2H5 | 64-66 |
| 114 | -CH3 | Br | O | O | -CH3 | |
| 115 | -CH3 | Cl | O | S | -CH3 | |
| 116 | -C(CH3)3 | Cl | O | S | C2H5 | 1,571 |
| 117 | -C(CH3)3 | Cl | O | S | -n-C3H7 | |
| 118 | -C(CH3)3 | Cl | O | S | -CH(CH3)2 | |
| 119 | -C(CH3)3 | Cl | O | S | -n-C4H9 | |
| 120 | -C(CH3)3 | Cl | O | S | -CH2-CH(CH3)2 | |
| 121 | -C(CH3)3 | Cl | O | S | -C(CH3)3 | |
| 122 | -C(CH3)3 | Cl | O | S | -n-C6H13 | |
| 123 | -C(CH3(3 | Cl | O | S | -C10H21 | 1,5115 |
| 124 | -C(CH3)3 | Cl | O | S | -CH2-CH2Cl | |
| 125 | -C(CH3)3 | Cl | O | S | -CH2-CH2-CH2Cl | |
| 126 | -C(CH3)3 | Cl | O | S | -CH2-CHCl-CH3 | |
| 127 | -C(CH3)3 | Cl | O | S | -CH2-CH=CH2 | |
| 128 | -C(CH3)3 | Cl | O | S | 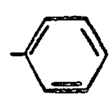 | 89-91 |
| 129 | -C(CH3)3 | Cl | O | S | 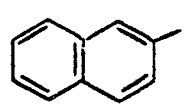 | |
| 130 | -C(CH3)3 | Cl | O | S | 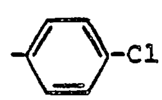 | |

| Verb.-Nr. | $R_1$ | $R_2$ | X | Y | Fp.(°C) $n_D^{23}$ |
|---|---|---|---|---|---|
| 131 | -C(CH3)3 | Cl | O | S | 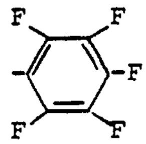 | |
| 132 | -C(CH3)3 | Cl | O | S | 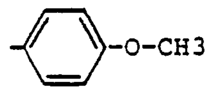 | |
| 133 | -C(CH3)3 | Cl | O | S | -CH2-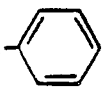 | |
| 134 | -CH2-CH2-O-CH3 | Cl | O | O | -n-C4H9 | 1,511 |
| 135 | -CH2-CH2-O-CH3 | Cl | O | O | -CH2-CH2-Br | 1,5281 |
| 136 | -CH2-CH2-O-CH3 | Cl | O | S | -C2H5 | 1,519 |
| 137 | -CH2-O-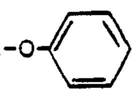 | Cl | O | O | -CH2-CH2Br | 100-103 |
| 138 | -CH2-CH2-O-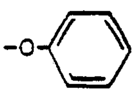 | Cl | O | O | -CH2-CH(CH3)2 | 1,514 |
| 139 | -CH2-O-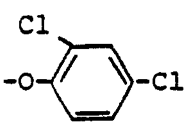 | Cl | O | S | -C2H5 | 60-62 |
| 140 | -CH2-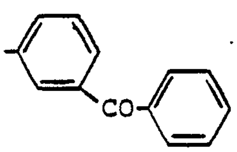 | Cl | O | O | -n-C4H9 | 1,542 |
| 141 | -CH2-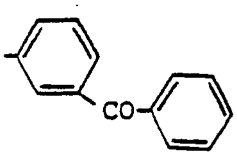 | Cl | O | S | -C2H5 | 1,551 |
| 142 | -CH2-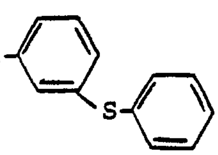 | Cl | O | S | -C2H5 | 1,574 |

| Verb.-Nr. | $R_1$ | $R_2$ | X | Y | $R_3$ | Fp. (°C) $n_D^{23}$ |
|---|---|---|---|---|---|---|
| 143 | -CH2-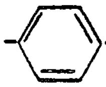-CH3 | Cl | O | S | -C2H5 | 1,531 |
| 144 | -CH2-CH2-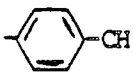-CH(CH3)2 | Cl | O | S | -C2H5 | 1,5285 |
| 145 | -CH2-CH2-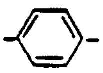- C(CH3)3 | Cl | O | O | -n-C4H9 | 1,508 |
| 146 | 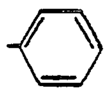 | Cl | O | O | -CH2-CH2-Br | 34-36 |
| 147 | 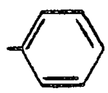 | Cl | O | S | -C2H5 | 176 |
| 148 | 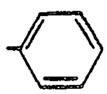-CH3 | Cl | O | O | -CH2-CH2Br | 85 |
| 149 | 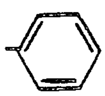-CH3 | Cl | O | O | -n-C4H9 | 1,509 |
| 150 | 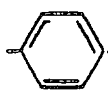-CH3 | Cl | O | S | -C2H5 | |
| 151 | 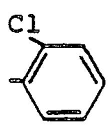 | Cl | O | O | -CH2-CH2Br | 30-32 |
| 152 | 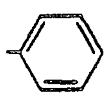-Cl | Cl | O | O | -CH2-CH2Br | 93 |
| 153 | 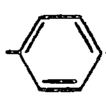-NO2 | Br | O | O | -CH2-CH2Br | 118-120 |
| 154 | -C(CH3)3 | Cl | S | O | -CH3 | |
| 155 | -C(CH3)3 | Cl | S | O | -C2H5 | |

| Verb.-Nr. | | $R_2$ | X | Y | $R_3$ | Fp.(°C) $n_D^{23}$ |
|---|---|---|---|---|---|---|
| 156 | -C(CH3)3 | Cl | S | O | -n-C3H7 | |
| 157 | -C(CH3)3 | Cl | S | O | -CH(CH3)2 | |
| 158 | -C(CH3)3 | Cl | S | O | -n-C4H9 | |
| 159 | -C(CH3)3 | Cl | S | O | -CH2-CH2Cl | |
| 160 | -C(CH3)3 | Cl | S | O | 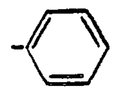 | 111-113 |
| 161 | -C(CH3)3 | Cl | S | O | 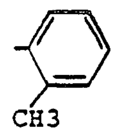 | |
| 162 | -C(CH3)3 | Cl | S | O | 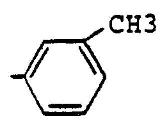 | |
| 163 | -C(CH3)3 | Cl | S | O | 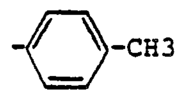 | 107 |
| 164 | -C(CH3)3 | Cl | S | O | 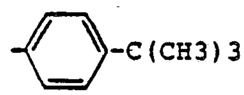 | |
| 165 | -C(CH3)3 | Cl | S | O | 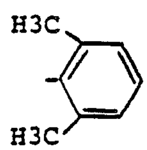 | |
| 166 | -C(CH3)3 | Cl | S | O | 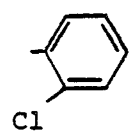 | |
| 167 | -C(CH3)3 | Cl | S | O | 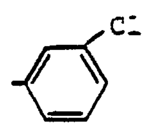 | |

| Verb.-Nr. | $R_1$ | $R_2$ | X | Y | $R_3$ | Fp. (°C) $n_D^{23}$ |
|---|---|---|---|---|---|---|
| 168 | -C(CH3)3 | Cl | S | O | 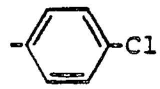 | 156 |
| 169 | -C(CH3)3 | Cl | S | O | 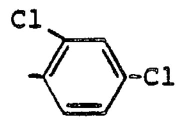 | |
| 170 | -C(CH3)3 | Cl | S | O | 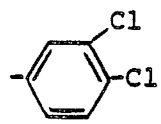 | |
| 171 | -C(CH3)3 | Cl | S | O | 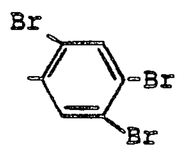 | |
| 172 | -C(CH3)3 | Cl | S | O | 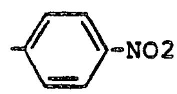 | |
| 173 | -C(CH3)3 | Cl | S | O | 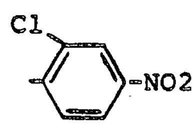 | |
| 174 | -C(CH3)3 | Cl | S | O | 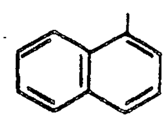 | |
| 175 | -C(CH3)3 | Cl | S | O | 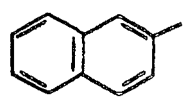 | |
| 176 | -C(CH3)3 | Cl | S | O | 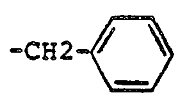 | |
| 177 | -C(CH3)3 | Cl | S | O | 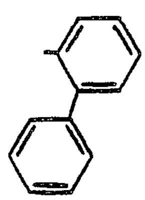 | |

| Verb.-Nr. | $R_1$ | $R_2$ | X | Y | $R_3$ | Fp. (°C) $n_D^{23}$ |
|---|---|---|---|---|---|---|
| 178 | -C(CH3)3 | Cl | S | O | 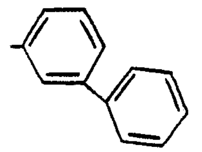 | |
| 179 | -C(CH3)3 | Cl | S | S | -CH3 | |
| 180 | -C(CH3)3 | Cl | S | S | -C2H5 | |
| 181 | -C(CH3)3 | Cl | S | S | -n-C3H7 | |
| 182 | -C(CH3)3 | Cl | S | S | -CH(CH3)2 | |
| 183 | -C(CH3)3 | Cl | S | S | -n-C4H9 | 1,5734 |
| 184 | -C(CH3)3 | Cl | S | S | 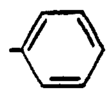 | 152-154 |
| 185 | -C(CH3)3 | Cl | S | S | 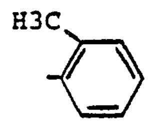 | |
| 186 | -C(CH3)3 | Cl | S | S | 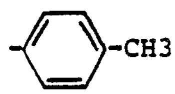 | |
| 187 | -C(CH3)3 | Cl | S | S | 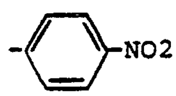 | |
| 188 | -C(CH3)3 | Cl | S | S | 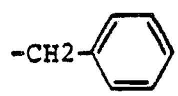 | |
| 189 | -C(CH3)3 | Cl | S | S | 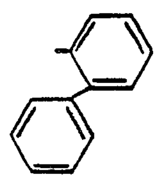 | |
| 190 | -C(CH3)3 | Cl | O | O | 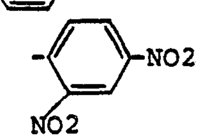 | |

| Verb.-Nr. | $R_1$ | $R_2$ | X | Y | $R_3$ | Fp. (°C) $n_D^{23}$ |
|---|---|---|---|---|---|---|
| 191 | -C(CH3)3 | Cl | O | O | 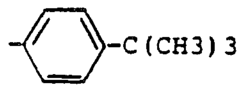 | |
| 192 | -C(CH3)3 | Cl | O | O | 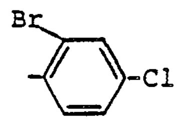 | |
| 193 | -C(CH3)3 | Cl | O | O | 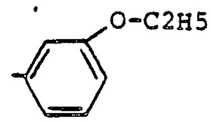 | |
| 194 | -C(CH3)3 | Cl | O | O | 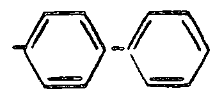 | |
| 195 | -C(CH3)3 | Cl | O | O | 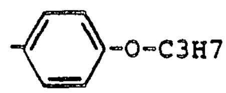 | |
| 196 | -C(CH3)3 | Cl | O | O | 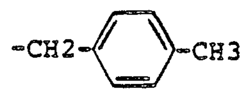 | |
| 197 | -C(CH3)3 | Cl | O | O | 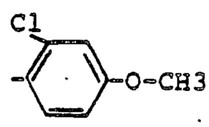 | |
| 198 | -C(CH3)3 | Cl | O | O | 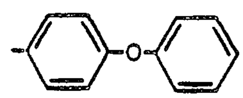 | |
| 199 | -C(CH3)3 | Cl | O | O | 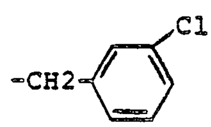 | |
| 200 | -C2H5 | Cl | O | S | -C2H5 | 1,553 |
| 201 | 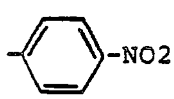 | Br | O | S | -C2H5 | 44-47 |
| 202 | 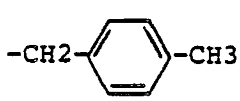 | Cl | O | O | -CH2-CH2Br | 1,592 |

| Verb.-Nr. | $R_1$ | $R_2$ | X | Y | $R_3$ | Fp. (°C) $n_D^{23}$ |
|---|---|---|---|---|---|---|
| 203 | -CH2-O- 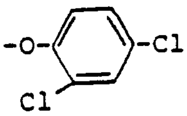 | Cl | O | O | -CH2-CH2Br | 1,582 |
| 204 | 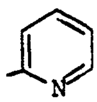 | Cl | O | O | -CH2-CH2Br | 34-36 |
| 205 | 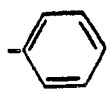 | Cl | S | S | 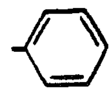 | 180-182 |
| 206 | 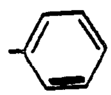 | Cl | S | S | -n-C4H9 | 198-200 |
| 207 | 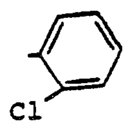 | Cl | S | S | 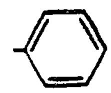 | 157 |
| 208 | -C(CH3)3 | Br | S | S | -n-C4H9 | 1,5757 |
| 209 | -C(CH3)3 | Br | O | O | -n-C3H7 | 1,573 |
| 210 | -C(CH3)3 | Br | O | S | -C2H5 | 1,5792 |
| 211 | -C(CH3)3 | Br | O | O | -CH2-CH=CH2 | 1,5789 |
| 212 | -CH2- 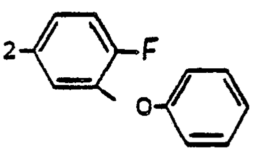 | Cl | O | O | -n-C3H7 | 1,579 |
| 213 | -CH2- 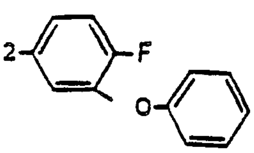 | Cl | O | O | -CH2-CH(C2H5)-(CH2)3-CH3 | 1,564 |
| 214 | -CH2- 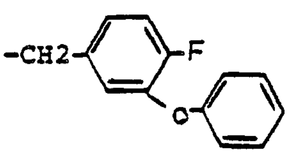 | Cl | O | O | -CH2-CH2Br | 1,568 |
| 215 | -C(CH3)3 | Cl | O | O | -CH2-C≡CH | 1,523 |

| Verb.-Nr. | $R_1$ | $R_2$ | X | Y | $R_3$ | Fp.(°C) $n_D^{23}$ |
|---|---|---|---|---|---|---|
| 216 | $-C(CH_3)_3$ | $CH_3$ | O | O | $-n-C_3H_7$ | |
| 217 | $-C(CH_3)_3$ | $-CH_2-CH_3$ | O | O | $-n-C_3H_7$ | |
| 218 | $-C(CH_3)_3$ | $-CH_2-CH_2-CH_3$ | O | O | $-n-C_3H_7$ | |
| 219 | $-C(CH_3)_3$ | $-(CH_2)_3-CH_3$ | O | O | $-n-C_3H_7$ | |
| 220 | $-C(CH_3)_3$ | $-C(CH_3)_3$ | O | O | $-n-C_3H_7$ | |
| 221 | 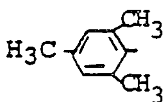 | Cl | O | O | $-n-C_3H_7$ | 130 |
| 222 | 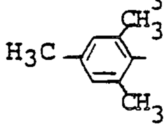 | Cl | O | S | $-C_2H_5$ | 116 |

## C. Biologische Beispiele

### Beispiel 1

Weizen und Gerste wurden im Gewächshaus in Plastiktöpfen bis zum 3-4 Blattstadium herangezogen und dann nacheinander mit den erfindungsgemäßen Verbindungen und den getesteten Herbiziden im Nachauflaufverfahren behandelt. Die Herbizide und die Verbindungen der Formel I wurden dabei in Form wäßriger Suspensionen bzw. Emulsionen mit einer Wasseraufwandmenge von umgerechnet 800 l/ha ausgebracht. 3-4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde.

Die Ergebnisse aus Tabelle 1 veranschaulichen, daß die erfindungsgemäßen Verbindungen starke Herbizidschäden an den Kulturpflanzen effektiv reduzieren können.

Selbst bei starken Überdosierungen des Herbizids werden bei den Kulturpflanzen auftretende schwere Schädigungen deutlich reduziert, geringere Schäden völlig aufgehoben. Mischungen aus Herbiziden und erfindungsgemäßen Verbindungen eignen sich deshalb in ausgezeichneter Weise zur selektiven Unkrautbekämpfung in Getreidekulturen.

### Beispiel 2

Reis wurde in Plastiktöpfen ausgesät und im Gewächshas unter optimalen Wachstumsbedingungen angezogen. Im 4-Blattstadium wurden die Pflanzen dann mit den Herbiziden und den Verbindungen der Formel I behandelt.

3 Wochen nach der Behandlung wurden die Pflanzen auf jede Art von Schädigung durch die Herbizide bonitiert, wobei insbesondere das Ausmaß der anhaltenden Wachstumshemmung und Ausdünnung betrachtet wurde.

Die Ergebnisse der Bonitur in Tabelle 1 zeigen, daß die Safener die Herbizidschäden an Reis effektiv reduzieren.

Mischungen aus Herbiziden und den erfindungsgemäßen Safenern eignen sich also zur selektiven Unkrautbekämpfung in Reis. Die herbizide Wirksamkeit der eingesetzten Herbizide gegen Schadpflanzen

wurde durch die Zugabe der erfindungsgemäßen Safener nicht beeinträchtigt; sie entsprach bei den verwendeten Aufwandmengen den Vergleichswerten, wie sie bei der Anwendung der Herbizide alleine erzielt wurde.

**Tabelle 1:**

Safenerwirkung der erfindungsgemäßen Verbindungen bei Weizen (TA), Gerste (HV) und Reis (OS); Wirkung in %

| Verbindung Nr. | Dosis kg a.i./ha | herbizide Wirkung TA | HV | OS |
|---|---|---|---|---|
| H | 2,0 | 75 | - | - |
| | 0,3 | - | - | 70 |
| | 0,2 | - | 85 | - |
| H + 58 | 2,0 + 2,5 | 10 | - | - |
| | 0,3 + 2,5 | - | - | 65 |
| | 0,2 + 2,5 | - | 40 | - |
| H + 60 | 2,0 + 2,5 | 20 | - | - |
| | 0,3 + 2,5 | - | - | 30 |
| | 0,2 + 2,5 | - | 40 | - |
| H + 39 | 2,0 + 2,5 | 30 | - | - |
| | 0,3 + 2,5 | - | - | 40 |
| | 0,2 + 2,5 | - | 40 | - |
| H + 68 | 2,0 + 2,5 | 40 | - | - |
| | 0,3 + 2,5 | - | - | 65 |
| | 0,2 + 2,5 | - | 30 | - |
| H + 21 | 2,0 + 2,5 | 60 | - | - |
| | 0,3 + 2,5 | - | - | 25 |
| | 0,2 + 2,5 | - | 30 | - |
| H + 16 | 2,0 + 2,5 | 25 | - | - |
| | 0,3 + 2,5 | - | - | 20 |
| | 0,2 + 2,5 | - | 20 | - |
| H + 20 | 2,0 + 2,5 | 40 | - | - |
| | 0,3 + 2,5 | - | - | 40 |
| | 0,2 + 2,5 | - | 20 | - |

Forts. Tabelle 1

| Verbindung Nr | Dosis kg a.i./ha | herbizide Wirkung TA | HV | OS |
|---|---|---|---|---|
| H + 116 | 2,0 + 2,5 | 30 | - | - |
| | 0,3 + 2,5 | - | - | 20 |
| | 0,2 + 2,5 | - | 20 | - |
| H + 111 | 2,5 + 2,5 | 30 | - | - |
| | 0,3 + 2,5 | - | - | 40 |
| | 0,2 + 2,5 | - | 20 | - |
| H + 42 | 2,0 + 2,5 | 25 | - | - |
| | 0,3 + 2,5 | - | - | 30 |
| | 0,2 + 2,5 | - | 20 | - |
| H + 40 | 2,0 + 2,5 | 25 | - | - |
| | 0,3 + 2,5 | - | - | 30 |
| | 0,2 + 2,5 | - | 20 | - |
| H + 151 | 2,0 + 2,5 | 65 | - | - |
| | 0,3 + 2,5 | - | - | 30 |
| | 0,2 + 2,5 | - | 85 | - |
| H + 51 | 2,0 + 2,5 | 40 | - | - |
| | 0,3 + 2,5 | - | - | 60 |
| | 0,2 + 2,5 | - | 30 | - |
| H + 112 | 2,0 + 2,5 | 40 | - | - |
| | 0,3 + 2,5 | - | - | 60 |
| | 0,2 + 2,5 | - | 30 | - |
| H + 204 | 2,0 + 2,5 | 25 | - | - |
| | 0,3 + 2,5 | - | - | 70 |
| | 0,2 + 2,5 | - | 70 | - |
| H + 19 | 2,0 + 2,5 | 20 | - | |
| | 0,3 + 2,5 | - | - | |
| | 0,2 + 2,5 | - | 70 | |

Forts. Tabelle 1

| Verbindung -Nr | Dosis kg a.i./ha | herbizide Wirkung TA | HV | OS |
|---|---|---|---|---|
| H + 17 | 2,0 + 2,5 | 40 | - | - |
| | 0,3 + 2,5 | - | - | 60 |
| | 0,2 + 2,5 | - | 30 | - |
| H + 75 | 2,0 + 2,5 | 30 | - | - |
| | 0,3 + 2,5 | - | - | 70 |
| | 0,2 + 2,5 | - | 30 | - |
| H + 104 | 2,0 + 2,5 | 30 | - | - |
| | 0,3 + 2,5 | - | - | 60 |
| | 0,2 + 2,5 | - | 30 | - |
| H + 29 | 2,0 + 2,5 | 10 | - | - |
| | 0,3 + 2,5 | - | - | 40 |
| | 0,2 + 2,5 | - | 15 | - |
| H + 85 | 2,0 + 2,5 | - | - | - |
| | 0,3 + 2,5 | - | - | 70 |
| | 0,2 + 2,5 | - | 40 | - |
| H + 18 | 2,0 + 2,5 | 10 | - | - |
| | 0,3 + 2,5 | - | - | 40 |
| | 0,2 + 2,5 | - | 20 | - |
| H + 101 | 2,0 + 2,5 | 25 | - | - |
| | 0,3 + 2,5 | - | - | 70 |
| | 0,2 + 2,5 | - | 40 | - |
| H + 138 | 2,0 + 2,5 | 40 | - | - |
| | 0,3 + 2,5 | - | - | 70 |
| | 0,2 + 2,5 | - | 40 | - |
| H + 84 | 2,0 + 2,5 | 30 | - | - |
| | 0,3 + 2,5 | - | - | 40 |
| | 0,2 + 2,5 | - | 30 | |

Forts. Tabelle 1

| Verbindung Nr. | Dosis kg a.i./ha | herbizide Wirkung TA | HV | OS |
|---|---|---|---|---|
| H + 74 | 2,0 + 2,5 | 25 | - | - |
| | 0,3 + 2,5 | - | - | 35 |
| | 0,2 + 2,5 | - | 28 | - |
| H + 144 | 2,0 + 2,5 | 30 | - | - |
| | 0,3 + 2,5 | - | - | 60 |
| | 0,2 + 2,5 | - | 80 | - |
| H + 200 | 2,0 + 2,5 | 40 | - | - |
| | 0,3 + 2,5 | - | - | 60 |
| | 0,2 + 2,5 | - | 60 | - |
| H + 145 | 2,0 + 2,5 | 75 | - | - |
| | 0,3 + 2,5 | - | - | 35 |
| | 0,2 + 2,5 | - | 75 | - |

Abkürzung:

H = Fenoxaprop-ethyl = (2-[4-(6-Chlorbenzoxazol-2-yl-oxy)-phenoxy]-propionsäureethylester

a.i. = Aktivsubstanz

## Ansprüche

1. Mittel zum Schutz von Kulturpflanzen gegen phytotoxische Nebenwirkungen von Herbiziden, dadurch gekennzeichnet, daß sie eine Verbindung der Formel I

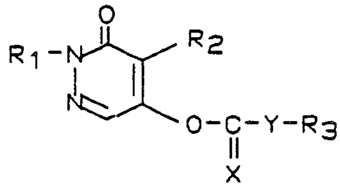

(I),

worin

$R_1$ $(C_1-C_6)$-Alkyl, $(C_1-C_3)$-Alkoxy-$(C_1-C_3)$-alkyl, Cyano-$(C_1-C_3)$-alkyl, Aryl, das ein- oder mehrfach durch $(C_1$-

$C_4$)-Alkyl, Halogen, ($C_1$-$C_3$)-Haloalkyl, ($C_1$-$C_4$)-Alkoxy, Phenyl, Phenoxy oder Nitro substituiert sein kann, Aryl-($C_1$-$C_3$)-alkyl, Aryloxy-($C_1$-$C_3$)-alkyl, wobei der Arylrest jeweils ein- oder mehrfach durch ($C_1$-$C_4$)-Alkyl, Halogen, ($C_1$-$C_3$)-Haloalkyl, ($C_1$-$C_3$)-Haloalkyl, ($C_1$-$C_4$)-Alkoxy, Aroyl, Arylthio oder Nitro substituiert sein kann, oder Pyridyl

$R_2$ Halogen oder ($C_1$-$C_4$)Alkyl,
X Sauerstoff oder Schwefel
Y Sauerstoff oder Schwefel und
$R_3$ ($C_1$-$C_{18}$)-Alkyl, das ein- oder mehrfach durch Halogen oder ($C_1$-$C_4$)-Alkoxy substituiert sein kann, ($C_2$-$C_4$)-Alkenyl, ($C_3$-$C_8$)-Cycloalkyl, Aryl, das ein- oder mehrfach durch ($C_1$-$C_3$)-Alkyl, Halogen, ($C_1$-$C_3$)-Alkoxy, ($C_1$-$C_3$)-Haloalkyl, ($C_1$-$C_3$)-Haloalkoxy oder Nitro substituiert sein kann, Aryl-($C_1$-$C_3$)-alkyl, Aryloxy-($C_1$-$C_3$)-alkyl, wobei der Arylteil jeweils durch Halogen oder Nitro substituiert sein kann, bedeuten in Kombination mit einem Herbizid enthalten.

2. Mittel gemäß Anspruch 1, dadurch gekennzeichnet, daß in Formel I
$R_1$ ($C_1$-$C_4$)-Alkyl oder Aryl, das durch Halogen substituiert sein kann, und
$R_3$ ($C_1$-$C_{12}$)-Alkyl, das ein- oder mehrfach durch Halogen substituiert sein kann, oder Benzyl
bedeuten.

3. Mittel gemäß Anspruch 1, dadurch gekennzeichnet, daß in Formel I
$R_1$ ($C_1$-$C_4$)-Alkyl
$R_2$ Chlor
X Sauerstoff und
$R_3$ ($C_1$-$C_8$)-Alkyl, das durch Halogen substituiert sein kann,
bedeuten.

4. Mittel gemäß einem oder mehreren der Ansprüche 1 bis 3, dadurch gekennzeichnet, daß als Herbizid eine Verbindung vom Typ der Phenoxyphenoxy- oder Heteroaryloxyphenoxycarbonsäure-($C_1$-$C_4$)-Alkyl-, ($C_2$-$C_4$)-Alkenyl- oder ($C_3$-$C_4$)-Alkinylester eingesetzt wird.

5. Mittel gemäß einem oder mehreren der Ansprüche 1 bis 4, dadurch gekennzeichnet, daß als Herbizid 2-(4-(6-Chlorbenzoxazol-2-yl-oxy)-phenoxy)-propionsäureethylester oder 2-(4-(6-Chlorbenzthiazol-2-yl-oxy)-phenoxy)-propionsäureethylester eingesetzt wird.

6. Mittel gemäß einem oder mehreren der Ansprüche 1 bis 5, dadurch gekennzeichnet, daß das Verhältnis Safener zu Herbizid 1 : 10 bis 10 : 1 beträgt.

7. Mittel gemäß einem oder mehreren der Ansprüche 1 bis 6, dadurch gekennzeichnet, daß das Verhältnis Safener zu Herbizid 2 : 1 bis 1 : 10 beträgt.

8. Verfahren zur Minderung der Phytotoxizität von Herbiziden gegenüber Kulturpflanzen, dadurch gekennzeichnet, daß man die Pflanzen, Pflanzensamen oder Anbauflächen mit einer wirksamen Menge einer Verbindung der Formel I vor, nach oder gleichzeitig mit dem Herbizid behandelt.

9. Verwendung von Verbindungen der Formel I zur Minderung der Phytotoxizität von Herbiziden gegenüber Kulturpflanzen.

10. Verbindungen der Formel I von Anspruch 1 (Ia), worin X oder Y oder X und Y Schwefel bedeuten.

11. Verbindungen der Formel I von Anspruch 1 (Ib), worin
X und Y = Sauerstoff,
$R_1$ = tert. Butyl
$R_2$ = Halogen oder ($C_1$-$C_4$)Alkyl
$R_3$ = ($C_1$-$C_4$)-Haloalkyl oder ($C_1$-$C_{12}$)-Alkyl bedeuten.

12. Verbindungen der Formel I von Anspruch I (Ib), worin
X und Y = Sauerstoff,
$R_1$ = tert. Butyl,
$R_2$ = Cl oder Br und
$R_3$ = ($C_1$-$C_4$)-Haloalkyl oder ($C_3$-$C_8$)-Alkyl bedeuten.

13. Verfahren zur Herstellung von Verbindungen der Formel I gemäß Ansprüchen 10 bis 12, dadurch gekennzeichnet, daß man eine Verbindung der Formel II

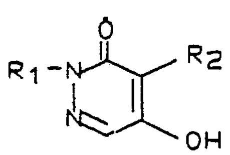

(II),

worin

$R_1$ und $R_2$ die Bedeutungen wie in Formeln Ia und Ib besitzen, mit einer Verbindung der Formel III

$$Z-\underset{\underset{X}{\|}}{C}-Y-R_3 \qquad (III),$$

worin

Z = Halogen bedeutet und X, Y und $R_3$ die Bedeutungen wie in Formeln Ia und Ib besitzen, in Gegenwart eines säurebindenden Mittels umsetzt.